(19) Europäisches Patentamt

European Patent Office

Office européen des brevets

(11) **EP 3 797 695 B1**

(12) **EUROPÄISCHE PATENTSCHRIFT**

(45) Veröffentlichungstag und Bekanntmachung des Hinweises auf die Patenterteilung:
**08.06.2022  Patentblatt 2022/23**

(21) Anmeldenummer: **20196929.2**

(22) Anmeldetag: **18.09.2020**

(51) Internationale Patentklassifikation (IPC):
**A61B 6/00** *(2006.01)*    **A61B 6/08** *(2006.01)*

(52) Gemeinsame Patentklassifikation (CPC):
**A61B 6/4405; A61B 6/08; A61B 6/4476; A61B 6/588**

(54) **AUTOMATISCHE POSITIONIERUNG EINER RÖNTGENQUELLE MITTELS SEGMENTIERUNG**

AUTOMATIC POSITIONING OF AN X-RAY SOURCE USING SEGMENTATION

POSITIONNEMENT AUTOMATIQUE D'UNE SOURCE DE RAYONS X PAR SEGMENTATION

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **30.09.2019  EP 19200519**

(43) Veröffentlichungstag der Anmeldung:
**31.03.2021  Patentblatt 2021/13**

(73) Patentinhaber: **Siemens Healthcare GmbH
91052 Erlangen (DE)**

(72) Erfinder:
• **Sutter, Sven-Martin
  91074 Herzogenaurach (DE)**
• **Lerch, Daniel
  91365 Weilersbach (DE)**

(56) Entgegenhaltungen:
**DE-A1-102009 013 572    DE-A1-102012 215 496
DE-A1-102013 219 137**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 3 797 695 B1

**Beschreibung**

Die Erfindung betrifft ein Verfahren zur automatischen Positionierung einer Röntgenquelle mittels Segmentierung.

Bei Aufnahmen mit einem frei positionierbaren Röntgendetektor, beispielsweise im Patientenbett zur Aufnahme der Lunge, ist es bisher notwendig, dass der Benutzer zur Einstellung bzw. Positionierung des Röntgenstrahlers bzw. der Röntgenquelle die Orientierung des Zentralstrahls und die räumliche Lage des Röntgendetektors, insbesondere nach Augenmaß, abschätzen muss. Zudem ist die direkte Sicht auf den Patienten und auf das eingeblendete Lichtfeld nur eingeschränkt möglich, da der Benutzer oft seitlich am Patienten und/oder an der Röntgenquelle steht. Durch diese Schätzung sind Ungenauigkeiten möglich, die zu einem Verlust an Bildqualität führen können.

Es ist Aufgabe der Erfindung, ein Verfahren zur automatischen Positionierung einer Röntgenquelle mittels Segmentierung, ein Röntgensystem, ein Computerprogrammprodukt und ein computerlesbares Medium anzugeben, welche eine genauere Ausrichtung der Röntgenquelle auf den Röntgendetektor ermöglichen.

Die Aufgabe wird erfindungsgemäß gelöst durch ein Verfahren zur automatischen Positionierung einer Röntgenquelle mittels Segmentierung nach Anspruch 1, ein Röntgensystem nach Anspruch 13, ein Computerprogrammprodukt nach Anspruch 14 und ein computerlesbares Medium nach Anspruch 15.

Die Erfindung betrifft ein Verfahren zur automatischen Positionierung einer Röntgenquelle eines medizinischen Röntgensystems mit einem mobilen Röntgendetektor aufweisend die Schritte des Bestimmens, des Erfassens, des Lokalisierens, des Ermittelns und des automatischen Positionierens. Im Schritt des Bestimmens wird eine Untersuchungsregion des Untersuchungsobjekts bestimmt. Im Schritt des Erfassens wird eine Position oder Positionierung und Lage des Untersuchungsobjekts und der Untersuchungsregion mittels eines optischen Positionsbestimmungssystems erfasst. Im Schritt des Lokalisierens wird die Untersuchungsregion lokalisiert. Im Schritt des Ermittelns werden ein Aufpunkt des Zentralstrahls der Röntgenquelle und eine Blendengröße der Röntgenquelle basierend auf der lokalisierten Untersuchungsregion ermittelt. Im Schritt des automatischen Positionierens wird die Röntgenquelle basierend auf dem Aufpunkt und der Blendengröße positioniert.

Die Erfindung betrifft die Anwendung in Verbindung mit einem Röntgensystem, insbesondere ein Radiographiesystem, mit einem mobilen Röntgendetektor. Der mobile Röntgendetektor kann auch als frei positionierbarer Röntgendetektor bezeichnet werden. Die Röntgenquelle kann insbesondere verfahrbar bzw. einstellbar sein. Die Röntgenquelle kann an einer verfahrbaren Wageneinheit montiert sein. Die Verbindung zwischen der Röntgenquelle und der Wageneinheit kann beispielsweise mittels eines sogenannten Knickarms mit mindestens einem Gelenk, mit der Wageneinheit verbunden sein. Die Röntgenquelle umfasst einen Zentralstrahl. Die Röntgenquelle kann ein Röntgenstrahlenbündel aussenden, welches mittels Blenden begrenzt werden kann. Die Größe des eingeblendeten Röntgenstrahlenbündels kann mittels der Blendengröße gewählt werden. Das eingeblendete Röntgenstrahlenbündel kann dann bei einer Röntgenaufnahme auf die Untersuchungsregion einfallen. Idealerweise kann die Ausdehnung des Röntgenstrahlenbündels auf der Auftrefffläche des Untersuchungsobjekts im Wesentlichen der Ausdehnung der Untersuchungsregion entsprechen. Vorteilhaft kann die Positionierung der Röntgenquelle automatisiert werden. Vorteilhaft kann eine verbesserte Positionierung erreicht werden. Vorteilhaft kann eine verbesserte Bildqualität erreicht werden.

Im Schritt des Bestimmens wird die Untersuchungsregion des Untersuchungsobjekts, insbesondere basierend auf einer Untersuchungsart bestimmt. Insbesondere kann der Nutzer einen Untersuchungsart oder/und eine Untersuchungsregion vorgeben bzw. eingeben. Die Eingabe kann beispielsweise einer Eingabeeinheit des Röntgensystems erfolgen. Alternativ kann die Röntgenquelle in Richtung der Untersuchungsregion, zumindest grob, ausgerichtet werden und die Untersuchungsregion "angepeilt" werden. Die Untersuchungsregion kann insbesondere vom Nutzer vorgegeben bzw. bestimmt werden. Die Untersuchungsregion kann als Untersuchungsbereich bezeichnet werden.

Im Schritt des Erfassens wird eine Position und eine Lage des Untersuchungsobjekts und die Untersuchungsregion mittels eines optischen Positionsbestimmungssystems erfasst. Der Schritt des Erfassens kann auch mit zwei Schritten beschrieben werden, das erste und das zweite Erfassen. Im Schritt des ersten Erfassens können eine Position und eine Lage eines Untersuchungsobjekts mittels eines optischen Positionsbestimmungssystems erfasst. Die Lage kann sich dabei insbesondere auf eine Anordnung im Raum, beispielsweise eine Orts- oder Raumkoordinate beziehen. Aus der Lage kann beispielsweise einen Abstand der Röntgenquelle zum Untersuchungsobjekt bzw. zur Untersuchungsregion abgeleitet werden. Die Position kann beispielsweise angeben, ob der Patient liegt, sitzt oder steht. Insbesondere kann eine Position des Untersuchungsobjekts in Zusammenhang mit der Nutzung von Positionierhilfen erfasst werden. Die Position kann beispielsweise angeben wie die Untersuchungsregion gelagert bzw. zur Röntgenquelle ausgerichtet ist. Die Position kann die Angabe einer Pose des Untersuchungsobjekts umfassen. Im Schritt des zweiten Erfassens kann die Untersuchungsregion mittels des optischen Positionsbestimmungssystems erfasst werden. Das Erfassen kann insbesondere eine im Wesentlichen dreidimensionale Aufnahme des Untersuchungsobjekts bzw. der Untersuchungsregion umfassen. Das optische Positionsbestimmungssystem kann insbesondere als 3D-Kamera ausgebildet sein. Das optische Bestimmungsinsbesondere infraroten, Bereich arbeiten. Im Schritt des Lokalisierens wird die Untersuchungsregion lokalisiert. Das Lokalisieren kann insbesondere die Position der Untersuchungsregion und die Ausdehnung der Untersuchungsregion umfassen. Der Schritt des Lokalisierens kann insbesondere auf der Erfassung der Untersuchungsregion als Teilbereich des Untersuchungsobjekts basieren. Im Schritt des Ermittelns wird ein Aufpunkt des Zentralstrahls der Röntgenquelle und eine Blendengröße der Röntgenquelle, insbesondere basierend auf der lokalisierten Untersuchungsregion, ermittelt. Der Aufpunkt kann einen Auftreffpunkt des Zentralstrahls auf der Patientenoberfläche bezeichnen. Im Schritt des automatischen Positionierens wird die Röntgenquelle basierend auf dem Aufpunkt und der Blendengröße positioniert. Die Röntgenquelle kann damit vorteilhaft möglichst ideal zur Untersuchungsregion und/oder dem Röntgendetektor ausgerichtet ist. Anschließend kann eine Röntgenaufnahme der Untersuchungsregion aufgenommen werden.

In der Röntgenanforderung bzw. dem Untersuchungsauftrag bzw. der Untersuchungsart wird das zu röntgende bzw. zu untersuchende Körperteil bzw. die Untersuchungsregion benannt. Es kann basierend auf dem angegebenen Körperteil auf ein vorbelegtes Organprogramm zurückgegriffen werden.

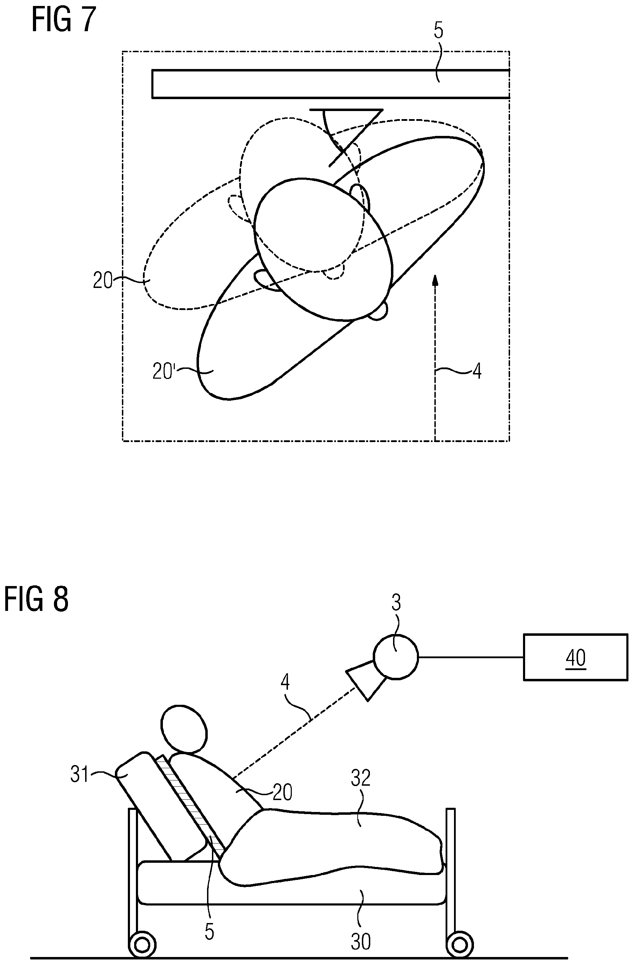

Mit Hilfe einer an der Röntgenquelle angebrachten Kamera bzw. eines optischen Positionsbestimmungssystems werden Bilder, insbesondere RGB und Tiefenbilder, bzw. Bilddaten aufgenommen bzw. erfasst. Aus diesen Bildern kann ein Avatar des Patienten bzw. Untersuchungsobjekts berechnet werden. Der Avatar kann auch als, insbesondere virtuelles, Patientenmodell bezeichnet werden. Dieser Avatar kann entsprechend der Röntgenanforderung bzw. der Untersuchungsart bzw. der Untersuchungsregion segmentiert werden. Im Schritt des Lokalisierens erfolgt eine Bildsegmentierung. Alternativ oder zusätzlich kann im Schritt des Ermittelns eine Bildsegmentierung erfolgen. Im Rahmen der Bildsegmentierung kann eine Zuordnung von Sementen zu Körperregionen und damit auch der Untersuchungsregion erfolgen. Aus der räumlichen Lage des Segments, insbesondere des Segments der Untersuchungsregion, kann die Position des Auftreffpunktes des Zentralstrahls bestimmt bzw. ermittelt werden. Die Größe des Strahlenfeldes der Röntgenquelle, insbesondere die Blendengröße, kann ebenfalls aus der Segmentierung bestimmt bzw. ermittelt werden. Dabei ist der Abstand zwischen Röntgenquelle und Objekt (SOD) aus den Messungen der Tiefenbilder bzw. einer Auswertung der Tiefeninformation bezüglich der Untersuchungsregion bekannt. Die Lage des Röntgendetektors kann aus der Lage des Untersuchungsobjekt bzw. der Untersuchungsregion bestimmt bzw. ermittelt werden. Zur Überprüfung der Winkellage des Röntgendetektors kann eine Information aus Winkelsensoren, die vom Röntgendetektor umfasst sein können, genutzt werden. Die Röntgenquelle kann automatisch in die korrekte Position verfahren werden, sodass der Zentralstrahl in Bezug zum Röntgendetektor entsprechend der Vorgaben eingestellt ist. Dabei wird angenommen, dass der Benutzer den Röntgendetektor unter dem zu röntgenden Körperteil bzw. hinter der Untersuchungsregion positioniert hat (vgl. Fig. 8).

Gemäß einem Aspekt der Erfindung sind im Schritt des Lokalisierens die Schritte des Erzeugens, des Segmentierens und des Lokalisierens umfasst. Im Schritt des Erzeugens wird ein Patientenmodell des Untersuchungsobjekts basierend auf der Erfassung des Untersuchungsobjekts bzw. der Untersuchungsregion mittels des optischen Positionsbestimmungssystems erzeugt. Die Erfassung kann auch als Aufnahme bezeichnet werden. Im Schritt des Segmentierens kann das Patientenmodell basierend auf der bestimmten Untersuchungsregion segmentiert werden. Im Schritt des Lokalisierens kann die Untersuchungsregion im Patientenmodell lokalisiert werden.

Das Patientenmodell kann insbesondere die Untersuchungsregion umfassen. Das Patientenmodell kann das gesamte Untersuchungsobjekt oder einen Teil des Untersuchungsobjekts umfassen. Das Patientenmodell kann insbesondere den Bereich des Sichtfelds des Positionsbestimmungssystems umfassen. Mit Hilfe von insbesondere von einer 3D-Kamera aufgenommen Bilddaten kann ein Avatar des Patienten bzw. des Untersuchungsobjekts erzeugt werden. Durch die Segmentierung des Avatars entsprechend der Vorgabe aus der Röntgenanforderung bzw. der Untersuchungsart kann die Lage des Röntgendetektors aus einer extrapolierten Kontur des Untersuchungsobjekts ermittelt werden. Optional kann die Lage des Röntgendetektors durch die Information aus den ggf. vom Röntgendetektor umfassten Winkelgebern bzw. - sensoren ermittelt werden. Die Röntgenquelle kann dann automatisch entsprechend der Lage des Röntgendetektors positioniert werden. Die Blende kann automatisch auf das zu röntgende Körperteil bzw. die Untersuchungsregion eingestellt werden.

Insbesondere eine 3D-Kamera kann als optisches Positionsbestimmungssystem an der Röntgenquelle montiert bzw. angeordnet sein. Idealerweise entspricht die optische Achse des optischen Positionsbestimmungssystems dem Zentralstrahl. Falls die optische Achse des optischen Positionsbestimmungssystems nicht mit dem Zentralstrahl übereinstimmt, können Kalibrationsmessungen durchgeführt werden, um eine genaue Lagebestimmung bzw. eine optimale Position der Röntgenquelle zu ermöglichen. Vorteilhaft kann der Arbeitsablauf bei der Untersuchung verbessert werden. Vorteilhaft kann eine verbesserte Bildqualität ermöglicht werden. Vorteilhaft kann ein Avatar des Untersuchungsobjekts zur Bestimmung der Lage des Röntgendetektors verwendet werden.

Gemäß einem Aspekt der Erfindung sind im Schritt des Lokalisierens die Schritte des Erzeugens und des Lokalisierens umfasst. Im Schritt des Erzeugens kann ein Patientenmodell des Untersuchungsobjekts oder ein Bilddatensatz des Untersuchungsobjekts basierend auf der Erfassung des Untersuchungsobjekts mittels des optischen Positionsbestimmungssystems erzeugt werden. Im Schritt des Lokalisierens kann die Untersuchungsregion mittels einem trainierten Auswertungsverfahren basierend auf einem maschinellen Lernverfahren lokalisiert werden. Das trainierte Auswertungsverfahren kann auch als trainierter Algorithmus bezeichnet werden.

Zunächst kann mittels des Positionsbestimmungssystems, insbesondere der Kamera oder mehreren Kameras, zuerst das Untersuchungsobjekt, beispielsweise im Untersuchungsraum, gesucht bzw. dessen Position bestimmt werden, insbesondere im Schritt des Erfassens. Das Untersuchungsobjekt kann auf dem Patiententisch oder einem Patientenbett liegen, oder vor einem Rasterwandgerät stehen, oder in einem Rollstuhl sitzen, oder neben dem Patiententisch sitzen.

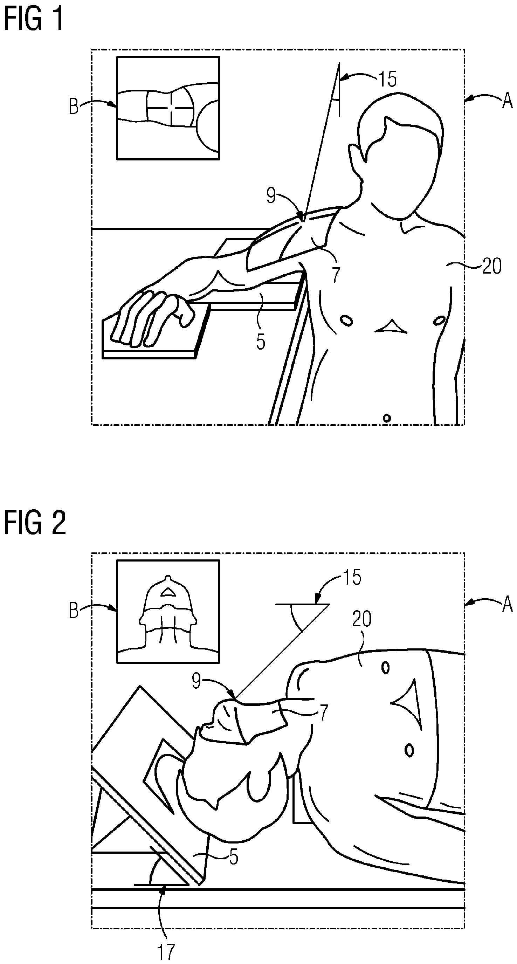

Aus der Untersuchungsart bzw. dem Untersuchungsprogramm bzw. dem Untersuchungsauftrag und den darin enthaltenen Untersuchungsdaten kann die Information über das zu röntgende Körperteil bzw. Organ, d.h. die Untersuchungsregion, extrahiert werden, insbesondere im Schritt des Bestimmens. Das Körperteil bzw. die Körperregion können nun mittels eines trainierten Algorithmus gesucht und deren Lage bestimmt werden. Damit kann der Aufpunkt des Zentralstrahls auf diesem Körperteil bzw. dem Organ bzw. der Untersuchungsregion bestimmt werden (vgl. Fig. 1 und 2). Zudem kann die Blendengröße bestimmt werden. In einer weiteren Ausführungsform kann die Untersuchungsregion vom Röntgensystem selbstständig gefunden werden und "angefahren", so dass die Untersuchungsregion vom Sichtfeld des optischen Positionsbestimmungssystems umfasst ist. Zudem kann mittels des optischen Positionsbestimmungssystems den Abstand zwischen Röntgenquelle und Röntgendetektor, die sogenannte SID, sowie die Dicke des Untersuchungsobjekts bestimmt werden.

Maschinelles Lernen im Sinne der Erfindung umfasst eine computer-implementierte Technik, bei der ein Algorithmus auf Basis von bestehenden Daten Muster bzw. Gesetzmäßigkeiten erkennt und unter Anwendung derselben in Bezug auf unbekannte, neue Daten eigenständig Lösungen ableitet. Voraussetzung für eine eigenständige Lösungsfindung ist eine Trainingsphase, in der ein Algorithmus des Maschinen-Lernens auf einen bekannten, definierten und zumeist sehr großen Datenbestand angewandt wird, um diejenigen Regeln bzw. Vorhersagen zu finden, die eine gewünschte Ausgabe bzw. ein gewünschtes Ergebnis erzielen. Das Training kann als überwachtes oder unüberwachtes Training ausgebildet sein, wobei in der ersten Variante dem Algorithmus Werte-Paare in Form von Eingabewerten und dazu gehörigen, korrekten Ausgabewerten präsentiert werden, wohingegen in der zweiten Variante der Algorithmus sich basierend auf den Eingabewerten eigenständig derart selber anpassen muss, dass er die korrekten Ausgabewerte liefert.

Besonders vorteilhaft ist der Algorithmus des Maschinen-Lernens als künstliches neuronales Netz ausgebildet. Ein künstliches neuronales Netz orientiert sich am Aufbau eines biologischen, neuronalen Netzes wie bspw. einem menschlichen Gehirn. Ein künstliches neuronales Netz umfasst zwischen einer Eingabe- und einer Ausgabeschicht bevorzugt eine Vielzahl von weiteren Schichten jeweils umfassend wenigstens einen Knoten. Jeder Knoten entspricht dabei einer Verarbeitungseinheit, analog einem biologischen Neuron. Knoten innerhalb einer Schicht des Netzes können über gerichtete Verbindungen (edges) mit Knoten anderer Schichten verbunden sein. Die Verbindungen definieren den Datenfluss innerhalb des Netzes. Jeder Knoten repräsentiert folglich eine Operation, die auf die Eingabedaten angewendet wird. Ferner verfügt jeder Knoten bzw. jede seiner Verbindungen über einen Gewichtungsparameter (weight). Über diesen Gewichtungsparameter wird der Einfluss bzw. die Wichtigkeit der Ausgabe eines Knotens als Eingabewert für einen Empfängerknoten definiert. In der Trainingsphase, die bevorzugt als überwachtes Lernen ausgeführt wird, 'lernt' das Künstliche Neuronale Netz anhand der Trainingsdaten die Gewichtungsparameter für alle Knoten bzw. Verbindungen und passt diese solange an, bis die Ausgabeschicht des Netzes die korrekten Ausgabewerte liefert.

Gemäß einem Aspekt der Erfindung beruht die Vorgehensweise ferner auf der Erkenntnis, dass ein trainierter Algorithmus des Maschinen-Lernens im Rahmen seines Trainings einen festen Zusammenhang zwischen Eingabewerten, hier in Form von Positionen oder Koordinaten und Eigenschaft des Untersuchungsobjekt bzw. Avatars, Untersuchungsart oder ähnlichem, und Ausgabewerten, beispielsweise in Form von Aufpunkt und Blendengröße, herstellt.

Besonders bevorzugt kann der Schritt des Lokalisierens mittels eines Algorithmus ausgeführt werden. In einem Schritt zum Finden des Untersuchungsobjekt bzw. der Untersuchungsregion an sich, kann ein trainierter Algorithmus verwendet werden. Besonders geeignet sind hierfür Algorithmen des sogenannten Deep Learnings, bspw. in Form eines 'convolutional neural networks', auch faltendes neuronales Netz genannt. Mit anderen Worten wird gemäß dieser Ausführung mittels eines Algorithmus des Maschinen-Lernens zunächst eine Merkmalsextraktion durchgeführt, und anschließend eine sogenannte Klassifikation, wobei die identifizierten Merkmale einer Position oder Lage des Untersuchungsobjekt o.ä. zugeordnet werden. Alternativ zu einem faltenden neuronalen Netz können auch Lange Kurzzeit-Gedächtnis (LSTM long short-term memory) Netze oder rekurrente neuronale Netze (RNN) zum Einsatz kommen, welche im Gegensatz zu den vorher genannten rückwärts gerichtete Feedback-Schleifen innerhalb der versteckten Netzschichten aufweisen.

Gemäß einem Aspekt der Erfindung kann ein nachfolgend beschriebenes künstliches neuronales Netz zum Einsatz kommen. Das neuronale Netz antwortet auf Eingabewerte zu einer Vielzahl von Eingangsknoten die angewendet werden, um eine oder eine Vielzahl von Ausgaben zu erzeugen. Das neuronale Netz lernt in diesem Ausführungsbeispiel, indem es die Gewichtungsfaktoren (weights) der einzelnen Knoten basierend auf Trainingsdaten anpasst. Mögliche Eingabewerte der Eingangsknoten können beispielsweise bestimmte Positionen oder Eigenschaften als Annotationen vorheriger Ausgaben sein, die zuvor aus bestehenden Datensätzen extrahiert wurden. Alternativ kann das neuronale Netz ausgebildet sein, auch die Merkmalsextraktion durchzuführen. Beliebige andere Eingabewerte können zur Anwendung kommen. Das neuronale Netz gewichtet die Eingabewerte basierend auf dem Lernprozess. Die Ausgabewerte des neuronalen Netzes entsprechen bevorzugt einem ermittelten Aufpunkt, einer Blendengröße und/oder einer Position der Röntgenquelle. Die Ausgabe kann über einen einzelnen oder eine Vielzahl von Ausgabeknoten erfolgen.

Das künstliche neuronale Netz umfasst bevorzugt eine versteckte Schicht, die eine Vielzahl von Knoten umfasst. Es können mehrere versteckte Schichten vorgesehen sein, wobei eine versteckte Schicht Ausgabewerte einer anderen versteckten Schicht als Eingabewerte verwendet. Die Knoten einer versteckten Schicht verrichten mathematische Operationen. Ein Ausgabewert eines Knotens $h_j$ entspricht dabei einer nichtlinearen Funktion f seiner Eingabewerte $x_i$ und der Gewichtungsfaktoren $w_i$. Nach dem Erhalt von Eingabewerten $x_i$, führt ein Knoten $h_j$ eine Summierung einer mit den Gewichtungsfaktoren $w_i$ gewichteten Multiplikation jedes Eingabewerts $x_i$ durch, wie durch folgende Funktion bestimmt:

$$h_j = f\left(\sum_i x_i \cdot w_{ij}\right).$$

Insbesondere wird ein Ausgabewert eines Knotens $h_j$ als Funktion f einer Knoten-Aktivierung, bspw. eine Sigmoidalfunktion oder eine lineare Rampenfunktion, gebildet. Die Ausgabewerte $h_j$ werden an den bzw. die Ausgabeknoten $o_j$ übertragen. Erneut wird eine Summierung einer gewichteten Multiplikation jedes Ausgabewertes $h_j$ als Funktion der Knoten-Aktivierung f berechnet:

$$o_j = f\left(\sum_i h_i \cdot w'_{ij}\right).$$

Das neuronale Netz kann ein Feedforward neuronales Netz sein, bei dem alle Knoten die Ausgabewerte einer vorherigen Schicht in Form ihrer gewichteten Summe als Eingabewerte verarbeiten. Selbstredend können erfindungsgemäß auch andere neuronale Netztypen zum Einsatz kommen, bspw. Feedback-Netze, bei denen ein Eingabewert eines Knotens $h_j$ gleichzeitig auch sein Ausgabewert sein kann.

Das neuronale Netz kann mittels einer Methode des überwachten Lernens trainiert werden, um Muster zu erkennen. Eine bekannte Vorgehensweise ist die Back-Propagation, die für alle Ausführungsbeispiele der Erfindung angewandt werden kann. Während des Trainings wird das neuronale Netz auf Trainings-Eingabewerten angewandt und muss entsprechende, vorher bekannte Ausgabewerte erzeugen. Iterativ werden mittlere quadratische Fehler (mean square error - "MSE") zwischen berechneten und erwarteten Ausgabewerten berechnet und einzelne Gewichtungsfaktoren so lange angepasst, bis die Abweichung zwischen berechneten und erwarteten Ausgabewerten unterhalb einer vorbestimmten Schwelle liegt.

Damit kann die Orientierung des Zentralstrahls und mit Hilfe des (vorgegebenen) Abstands zwischen Röntgenquelle und Untersuchungsregion bzw. -objekt und basierend auf einem deep learning basierten Training die Position der Röntgenquelle relativ zum Körperteil bzw. Organ bzw. der Untersuchungsregion festgelegt werden. Ferner kann ein sogenanntes Skeletal Tracking als trainiertes Auswertungsverfahren verwendet werden.

Der Zentralstrahl kann nun auf den Aufpunkt ausgerichtet werden und im richtigen bzw. optimalen Winkel im Raum, in Fig. 1 beispielsweise 15 Grad, bzw. zur Orientierung oder Lage des Patienten positioniert werden. Die Blende kann auf die richtige bzw. optimale Größe eingestellt werden.

Gemäß einem Aspekt der Erfindung wird eine Lage des Röntgendetektors basierend auf der Lage des Untersuchungsobjekts, des Patientenmodells oder des lokalisierten Untersuchungsbereichs ermittelt.

In einem nachfolgenden Schritt kann die genaue Lage des Röntgendetektors gesucht bzw. bestimmt werden. Der Röntgendetektor wird in dem "Zielbereich" des Zentralstrahls erwartet. Die etwaigen Winkelsensoren des Röntgendetektors können die Orientierung einer ersten Ebene im Raum bereitstellen bzw. ermitteln, in der sich auch der Röntgendetektor befindet. Die Lage der ersten Ebene kann mit einer zweiten Ebene verglichen werden, die sich durch eine an die "Rückseite" bzw. die dem Röntgendetektor zugewandte Seite des Avatars gefittete bzw. angenäherte Ebene ergibt. Die Winkellage des Röntgendetektors sollte dabei von der vom Zentralstrahl vorgegebenen Orientierung und den von der jeweiligen Untersuchung bzw. Untersuchungsart vorgegebenen Winkeln im Wesentlichen entsprechen.

Zum Finden des Röntgendetektors bzw. zur Lagebestimmung des Röntgendetektors können beispielsweise optische Verfahren genutzt werden. Beispielsweise können einfache Muster im sichtbaren Licht verwendet werden. Alternativ oder zusätzlich können Ortungsverfahren basierend auf Ultraschall oder Radar oder elektromagnetische Ortungsverfahren zur Lagebestimmung des Röntgendetektors verwendet werden.

Gemäß einem Aspekt der Erfindung wird die Lage des Röntgendetektors mittels Winkelsensoren des Röntgendetektors ermittelt. Die Lage des Röntgendetektors kann ggf. zusätzlich zu anderen Verfahren, beispielsweise optischen Verfahren, mittels Winkelsensoren oder anderen Sensoren verbessert ermittelt werden. Es können alternativ oder zusätzlich optische Marker, beispielsweise zur Nutzung mit infrarotem Licht, zur Positionsbestimmung des Röntgendetektors genutzt werden.

In einer Ausführungsform kann auch eine Kombination von Ortungsverfahren genutzt werden, beispielsweise kann der Röntgendetektor zunächst optisch gefunden bzw. dessen Lage ermittelt werden. Falls der Röntgendetektor anschließend bewegt wird und dann ggf. vollständig hinter den Patienten bzw. den Untersuchungsbereich geschoben wird, kann dies Positions- bzw. Lageänderung mit Hilfe von zusätzlich eingebauten Sensoren, z.B. Beschleunigungssensoren, ermittelt werden. Die so ermittelte Lages des Röntgendetektors kann mit der angenommenen Lage verglichen werden, um gegebenenfalls die Ausrichtung des Röntgendetektors zum Zentralstrahl zu optimieren.

Gemäß einem Aspekt der Erfindung wird der lokalisierte Untersuchungsbereich auf einer Anzeigeeinheit oder mittels eines auf das Untersuchungsobjekt projizierten Lichtfelds angezeigt.

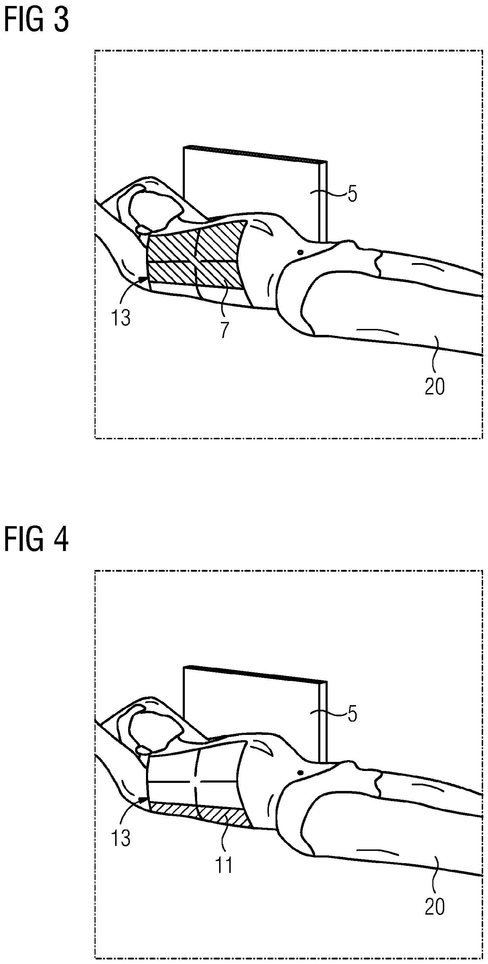

Sobald die im Wesentlichen genaue Lage des Röntgendetektors im Raum bekannt ist, kann angezeigt werden, ob der eingeblendete Bereich des Röntgenstrahlenbündels vollständig auf dem aktiven Bereich des Röntgendetektors abgebildet wird. Bevorzugt kann (nur) der auf den aktiven Bereich treffende Abbildungsbereich in einem virtuellen Lichtfeld, beispielsweise auf einem Bildschirm oder einem Touch User Interface oder mittels eines projizierten Lichtfelds markiert und dargestellt werden (vgl. Fig. 3 bis 5). Es kann zudem oder alternativ der Bereich hervorgehoben werden, welcher nicht auf den aktiven Bereich des Röntgendetektors abgebildet werden kann.

Gemäß einem Aspekt der Erfindung wird ein Vorschlag zur Positionskorrektur des Untersuchungsobjekts ausgegeben.

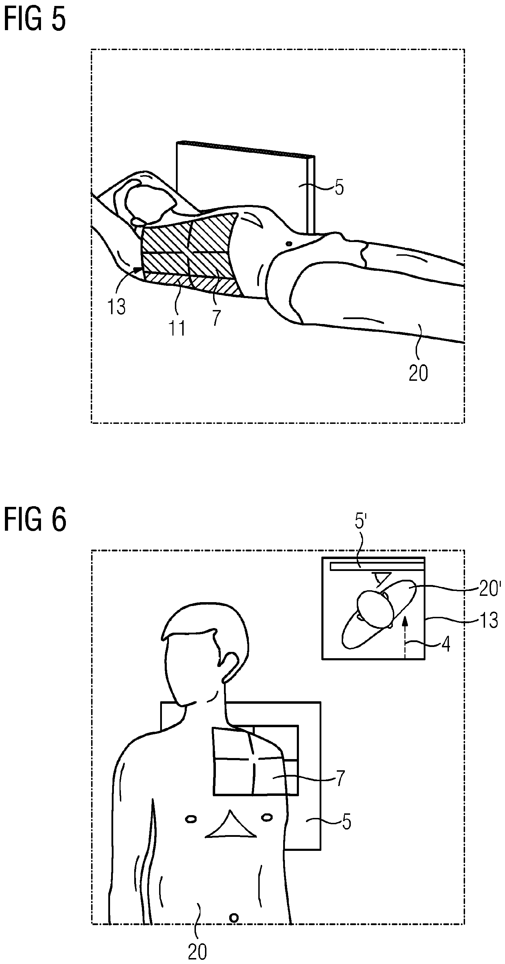

In einer weiteren Ausführungsform kann nach dem Positionieren des Patienten bzw. des Untersuchungsobjekt, insbesondere bei bestimmten Aufnahmen bzw. Untersuchungsarten, zusätzlich oder alternativ die vorgesehene Position des Patienten bzw. des Untersuchungsobjekts bzw. der Untersuchungsregion überprüft werden. Beispielsweise kann der Winkel zwischen Schulterachse und Röntgendetektor mit Hilfe der 3D-Information gemessen, angezeigt und mit einem Sollwert, einem sogenannten Lehrbuch-Wert, verglichen werden (vgl. Fig. 6 und 7). Es kann eine Soll-Ist-Darstellung angezeigt werden. Es kann ein Abgleich zur optimalen Ausrichtung des Untersuchungsobjekts durchgeführt werden. Ferner kann eine, insbesondere virtuelle oder mittels Projektionen geführte, Benutzerführung zur Patientenpositionierung vorgesehen sein.

Der Untersuchungsworkflow kann vorteilhaft weitgehend beibehalten werden. Der Untersuchungsworkflow kann vorteilhaft in den einzelnen Phasen der Untersuchung bzw. Aufnahme schrittweise unterstützt werden.

Die Position der Röntgenquelle kann auf den zu röntgenden Bereich bzw. die Untersuchungsregion fokussiert sein anstatt auf die Mitte des Röntgendetektors. Vorteilhaft kann die Einblendung des Röntgenstrahlenbündels optimiert werden. Vorteilhaft kann die Patientendosis reduziert werden. Vorteilhaft kann eine Optimierung der Patientenposition ermöglicht werden.

Auf Grund der Annahme, dass der Röntgendetektor in einem im Wesentlichen bekannten Bereich erwartet werden kann, können die Anforderungen an das Röntgendetektor-Ortungs-System zumindest für den Entfernungsbereich reduziert werden. Vorteilhaft kann das System kostengünstig ausgestaltet sein.

Gemäß einem Aspekt der Erfindung wird eine Bewegung des Untersuchungsobjekts mittels des optischen Positionsbestimmungssystems erfasst. Nach Abklingen der Bewegung kann die Positionierung der Röntgenquelle nachkorrigiert werden, indem der Aufpunkt und der Richtungsvektor der Röntgenquelle erneut berechnet und justiert bzw. eingestellt wird. In einer Ausführungsform kann der Aufpunkt und die Blendengröße iterativ bestimmt werden, insbesondere unter Einfluss von Patientenbewegungen. Durch den Aufpunkt und ggf. durch einen durch die Untersuchungsart oder Untersuchungsregion vorgegebenen bzw. vorbestimmten Winkel kann der Zentralstrahl der Röntgenquelle entlang des Richtungsvektors ausgerichtet werden. Die Erfassung der Bewegung kann in einer weiteren Ausführungsform für einen Atemtrigger verwendet werden, wobei mehrere Atemzyklen erfasst und ausgewertet werden, so dass die Röntgenaufnahme in einer Ruhephase oder bewegungsarmen Phase in einem nachfolgenden Atemzyklus, basierend auf den ausgewerteten Atemzyklen, aufgenommen werden kann. Beispielsweise wird bei Lungenaufnahmen typischerweise bei voller Inspiration die Röntgenstrahlung ausgelöst und die Röntgenaufnahme durchgeführt. Vorteilhaft kann die Bildqualität verbessert werden.

Gemäß einem Aspekt der Erfindung wird eine erneute Erfassung und eine erneute Lokalisierung der Untersuchungsregion durchgeführt und die Positionierung der Röntgenquelle basierend auf der erneuten Lokalisierung korrigiert.

In einer weiteren Ausführungsform können kleine Bewegungen des Untersuchungsobjekts, beispielsweise Rucker, Verschnaufer oder Huster, nach Abklingen der Bewegung nachkorrigiert werden, wobei der Aufpunkt und ggf. daraus der Richtungsvektor der Röntgenquelle neu berechnet und justiert werden kann. Anschließend kann eine Anzeige erfolgen, um den Benutzer über die Korrektur zu informieren. Vorteilhaft kann eine Korrektur nach einer Patientenbewegung durchgeführt werden, so dass die Bildqualität verbessert werden kann.

Gemäß einem Aspekt der Erfindung wird der Zentralstrahl der Röntgenquelle basierend auf der Untersuchungsregion oder einer Untersuchungsart in einem vorbestimmten Winkel zum Untersuchungsregion ausgerichtet. Je nach Untersuchungsart oder Untersuchungsregion kann ein Winkel des Zentralstrahls zur Untersuchungsregion vorbestimmt sein. Bei der Ermittlung des Aufpunkts und der anschließenden automatischen Positionierung kann dieser Winkel berücksichtigt werden.

Gemäß einem Aspekt der Erfindung wird der Zentralstrahl der Röntgenquelle im Wesentlichen senkrecht zum Röntgendetektor ausgerichtet. Die, insbesondere übliche, Ausrichtung des Zentralstrahls kann entlang der Flächennormalen des Röntgendetektors ausgebildet sein. Vorteilhaft kann eine gute Bildqualität erreicht werden.

Gemäß einem Aspekt der Erfindung weicht der Zentralstrahl der Röntgenquelle von einer im Wesentlichen senkrechten Ausrichtung zum Röntgendetektor ab und eine Bildkorrektur einer Röntgenaufnahme wird hinsichtlich der Abweichung durchgeführt. Falls der Zentralstrahl von der Flächennormalen des Röntgendetektors abweicht, kann eine Bildkorrektur durchgeführt werden, um die Bildqualität vorteilhaft zu verbessern.

In einer weiteren Ausführungsform können die beiden Anforderungen, nämlich dass die Röntgenquelle in einem optimalen Winkel zum (freien) Röntgendetektor, also i.d.R. senkrecht ausgerichtet ist, und dass die Röntgenquelle in einem optimalen Winkel zur anatomischen Struktur bzw. zur Untersuchungsregion, betrachtet werden. Der optimale Winkel zur anatomischen Struktur kann insbesondere bei orthopädischen Untersuchungen, beispielsweise von Gelenkspalten, von Bedeutung sein. Die beiden Anforderungen können nicht in allen Fällen gleichzeitig und gleich gut erfüllt werden, beispielsweise weil der Patient dazu unpassend auf dem Patientenbett oder einem Kissen gelagert ist. Beispielsweise kann der (freie) Röntgendetektor nicht derart angeordnet werden, dass die Röntgenquelle optimal bzw. ideal zur anatomischen Struktur und gleichzeitig zum Röntgendetektor ausgerichtet ist. In diesen Fällen, zumindest wenn kein Raster am Röntgendetektor verwendet wird, kann die Röntgenquelle bevorzugt optimal zur anatomischen Struktur ausgerichtet werden. Vorteilhaft kann die Ausrichtung der Röntgenquelle zur Untersuchungsregion auch dann zuverlässig bestimmt werden, wenn der Röntgendetektor hinter dem Untersuchungsobjekt nicht sichtbar ist. Eine ggf. ungenaue Ausrichtung gegenüber dem Röntgendetektor, welche in Form einer Verzerrung der Aufnahme sichtbar werden kann, kann digital und nachträglich korrigiert werden. Die Korrektur kann basierend auf den eingestellten Positionsparametern der Röntgenquelle nämlich Lage und Winkel, und denen des Röntgendetektors, basierend auf Messungen von Lagesensoren bzw. Gyrosensoren, erfolgen. Die Lage der anatomischen Struktur kann durch das optische Positionsbestimmungssystem, beispielsweise eine 3D-Kamera, bestimmt werden. Die Winkelabweichung des Körperteils bzw. der Untersuchungsregion kann daraus berechnet werden. Die Abweichung kann zur Korrektur bzw. Entzerrung der zweidimensionalen (Röntgen-)Aufnahme verwendet werden.

Die Erfindung betrifft ferner ein Röntgensystem aufweisend Mittel zum Ausführen eines erfindungsgemäßen Verfahrens. Das Röntgensystem kann insbesondere ein (mobiles) Radiographiesystem sein. Das Röntgensystem weist eine Röntgenquelle mit einem optischen Positionsbestimmungssystem und einen mobilen Röntgendetektor auf. Die optische Achse des Positionsbestimmungssystems kann idealerweise dem Zentralstrahl der Röntgenquelle entsprechen. Andernfalls kann eine Abweichung vom Zentralstrahl beim Ermitteln berücksichtigt werden.

Die Erfindung betrifft ferner ein Computerprogrammprodukt mit einem Computerprogramm, welches direkt in eine Speichereinrichtung einer Steuereinrichtung eines Röntgensystems ladbar ist, mit Programmabschnitten, um alle Schritte eines erfindungsgemäßen Verfahrens auszuführen, wenn das Computerprogramm in der Steuereinrichtung des Röntgensystems ausgeführt wird.

Die Erfindung betrifft ferner ein computerlesbares Medium, auf welchem von einer Rechnereinheit einlesbare und ausführbare Programmabschnitte gespeichert sind, um alle Schritte eines erfindungsgemäßen Verfahrens auszuführen, wenn die Programmabschnitte von dem Röntgensystem ausgeführt werden.

Nachfolgend werden Ausführungsbeispiele der Erfindung anhand von Zeichnungen näher erläutert. Hierbei zeigt:

FIG 1 eine schematische Darstellung eines erfindungsgemäß ermittelten Aufpunkts und einer eingeblendeten Untersuchungsregion in einer ersten Ausführungsform;

FIG 2 eine schematische Darstellung eines erfindungsgemäß ermittelten Aufpunkts und einer eingeblendeten Untersuchungsregion in einer zweiten Ausführungsform;

FIG 3 eine schematische Darstellung einer erfindungsgemäßen Anzeige des eingeblendeten Untersuchungsbereichs relativ zum Untersuchungsobjekt und dem Röntgendetektor in einer ersten Ausführungsform;

FIG 4 eine schematische Darstellung einer erfindungsgemäßen Anzeige des eingeblendeten Untersuchungsbereichs relativ zum Untersuchungsobjekt und dem Röntgendetektor in einer zweiten Ausführungsform;

FIG 5 eine schematische Darstellung einer erfindungsgemäßen Anzeige des eingeblendeten Untersuchungsbereichs relativ zum Untersuchungsobjekt und dem Röntgendetektor in einer dritten Ausführungsform;

FIG 6 eine schematische Darstellung einer erfindungsgemäßen Überprüfung einer Lage oder Position des Untersuchungsobjekts in einer ersten Ausführungsform;

FIG 7 eine schematische Darstellung einer erfindungsgemäßen Überprüfung einer Lage oder Position des Untersuchungsobjekts in einer zweiten Ausführungsform;

FIG 8 eine schematische Darstellung eines erfindungsgemäßen Röntgensystems; und

FIG 9 eine schematische Darstellung eines erfindungsgemäßen Verfahrens.

Die Fig. 1 zeigt eine beispielhafte Ausführung eines erfindungsgemäß ermittelten Aufpunkts 9 und einer eingeblendeten Untersuchungsregion 7 in einer ersten Ausführungsform. Zunächst ist eine Seitenansicht A dargestellt. Das Untersuchungsobjekt 20 sitzt neben einem Tisch und der Arm mit der Untersuchungsregion 7 ist mittels Lagerungshilfe auf dem Tisch gelagert. Der Röntgendetektor 5 ist unterhalb der Untersuchungsregion 7 auf dem Tisch gelagert. Die Untersuchungsregion 7 liegt im Bereich der Schulter. Ein Winkel 15 ist zwischen der Raumvertikalen und dem Zentralstrahl vorgegeben, dieser beträgt beispielsweise 15 Grad. Zudem ist der mittels der eingestellten Blendengröße eingeblendete Bereich als Untersuchungsregion 7 und der Aufpunkt 9 dargestellt. In der Draufsicht B ist die Untersuchungsregion ebenfalls dargestellt. Die Draufsicht B kann beispielsweise von einer Anzeigeeinheit angezeigt werden. Die Draufsicht B kann insbesondere eine Draufsicht mit Blick entlang der optischen Achse bzw. des Zentralstrahls sein.

Die Fig. 2 zeigt eine beispielhafte Ausführung eines erfindungsgemäß ermittelten Aufpunkts 9 und einer eingeblendeten Untersuchungsregion 7 in einer zweiten Ausführungsform. Zunächst ist eine Seitenansicht A dargestellt. Das Untersuchungsobjekt 20 liegt auf dem Rücken mit einem überstreckten Kopf. Die Untersuchungsregion 7 liegt im Bereich des Gesichts. Der Röntgendetektor 5 ist mittels einer Haltehilfe auf dem Tisch in einem Winkel 17 zur Tischoberfläche gelagert. Ein Winkel 15 ist zwischen der Raumhorizontalen und dem Zentralstrahl vorgegeben, dieser beträgt beispielsweise 45 Grad. Zudem ist der mittels der eingestellten Blendengröße eingeblendete Bereich als Untersuchungsregion 7 und der Aufpunkt 9 dargestellt. In der Draufsicht B ist die Untersuchungsregion ebenfalls dargestellt. Die Draufsicht B kann beispielsweise von einer Anzeigeeinheit angezeigt werden.

Die Fig. 3 zeigt eine beispielshafte Ausführung einer Anzeige des eingeblendeten Untersuchungsbereichs 7 relativ zum Untersuchungsobjekt 20 und dem Röntgendetektor 5 in einer ersten Ausführungsform. Die Anzeige kann beispielsweise mittels eines, ggf. virtuellen, Lichtfelds 13 erfolgen. Das Lichtfeld 13 kann auf das Untersuchungsobjekt 20 projiziert werden. Alternativ kann ein virtuelles Lichtfeld 13 auf einer Anzeigeeinheit auf einem Kamerabild überlagert werden. Sobald die im Wesentlichen genaue Lage des Röntgendetektors 5 im Raum bekannt ist, kann angezeigt werden, ob der eingeblendete Bereich des Röntgenstrahlenbündels vollständig auf dem aktiven Bereich des Röntgendetektors abgebildet wird. Bevorzugt kann nur der auf den aktiven Bereich treffende Abbildungsbereich in einem virtuellen Lichtfeld 13, beispielsweise auf einem Bildschirm oder einem Touch User Interface oder mittels eines projizierten Lichtfelds markiert und dargestellt werden. Es wird der eingeblendete Bereich des Untersuchungsbereichs 7, der auf dem aktiven Bereich des Röntgendetektors abgebildet wird, dargestellt.

Die Fig. 4 zeigt eine beispielshafte Ausführung einer Anzeige des eingeblendeten Untersuchungsbereichs 7 relativ zum Untersuchungsobjekt 20 und dem Röntgendetektor 5 in einer zweiten Ausführungsform. Es wird der Bereich 11 gezeigt, welcher außerhalb des aktiven Bereichs des Röntgendetektors liegt.

Die Fig. 5 zeigt eine beispielshafte Ausführung einer Anzeige des eingeblendeten Untersuchungsbereichs 7 relativ zum Untersuchungsobjekt 20 und dem Röntgendetektor 5 in einer dritten Ausführungsform. Es wird sowohl der eingeblendete Bereich des Untersuchungsbereichs 7, der auf dem aktiven Bereich des Röntgendetektors abgebildet wird, als auch der Bereich 11 gezeigt werden, welcher außerhalb des aktiven Bereichs des Röntgendetektors liegt. Die Bereiche 7 und 11 werden unterscheidbar angezeigt, beispielsweise mittels unterschiedlicher Farben.

Die Fig. 6 zeigt eine beispielshafte Ausführung der Überprüfung einer Lage oder Position des Untersuchungsobjekts 20 in einer ersten Ausführungsform. Es wird nach dem Positionieren des Untersuchungsobjekt, insbesondere bei bestimmten Aufnahmen bzw. Untersuchungsarten, die vorgesehene Lage oder Position des Untersuchungsobjekts bzw. der Untersuchungsregion überprüft. Das Untersuchungsobjekt 20 ist stehend vor dem Röntgendetektor 5 in einem Winkel positioniert. Der Untersuchungsbereich 7 ist eingeblendet. In einer Draufsicht ist eine Soll-Position, beispielsweise auf einer Anzeigeeinheit, dargestellt. Das Untersuchungsobjekt 20' ist in einem vorbestimmten angezeigten Winkelbereich idealerweise vor dem Röntgendetektor 5' zu positionieren. Der Zentralstrahl 4 wird ebenfalls angezeigt.

Die Fig. 7 zeigt eine beispielshafte Ausführung der Überprüfung einer Lage oder Position des Untersuchungsobjekts 20 in einer zweiten Ausführungsform. Es wird ein Vorschlag zur Positionskorrektur des Untersuchungsobjekts 20, beispielsweise auf einer Anzeigeeinheit, ausgegeben. Beispielsweise wird der Winkel zwischen Schulterachse und Röntgendetektor mit Hilfe der 3D-Information gemessen, angezeigt und mit einem Sollwert, einem sogenannten Lehrbuch-Wert, verglichen. Es wird eine Soll-Ist-Darstellung angezeigt. Es kann ein Abgleich zur optimalen Ausrichtung des Untersuchungsobjekts 20 durchgeführt werden. Ferner kann eine, insbesondere virtuelle oder mittels Projektionen geführte, Benutzerführung zur Patientenpositionierung vorgesehen sein. Die Soll-Position des modelhaften Untersuchungsobjekts 20' wird ggf. mit einer Winkelangabe angezeigt. Die Ist-Position des Untersuchungsobjekts 20 wird überlagert dargestellt. Eine farbliche Codierung der Darstellung des Untersuchungsobjekts kann eine korrekte (grün) oder inkorrekte (rot) Positionierung oder Lage des Untersuchungsobjekts 20 anzeigen. Zusätzlich kann der Zentralstrahl 4 zur Orientierung angezeigt werden.

Die Fig. 8 zeigt eine beispielshafte Ausführung eines erfindungsgemäßen Röntgensystems zum Durchführen eines erfindungsgemäßen Verfahrens. Das Röntgensystem weist eine Röntgenquelle 3 mit einem optischen Positionsbestimmungssystem, und einen mobilen Röntgendetektor 5 auf. Das Untersuchungsobjekt 20 ist beispielshaft in einem Patientenbett 30 mittels Kissen 31 gelagert. Die Röntgenquelle 3 mit dem Zentralstrahl 4 strahlt beispielhaft auf den Rumpf des Untersuchungsobjekts 20 ein. Der Röntgendetektor 5 befindet sich beispielhaft hinter dem Untersuchungsobjekt 20 entlang des Rückens. Der Röntgendetektor 5 ist zwischen dem Kissen 31 und dem Untersuchungsobjekt 20 angeordnet. Das Untersuchungsobjekt ist mit einer Decke 32 zugedeckt. Das Röntgensystem weist ferner eine Rechnereinheit 40 auf.

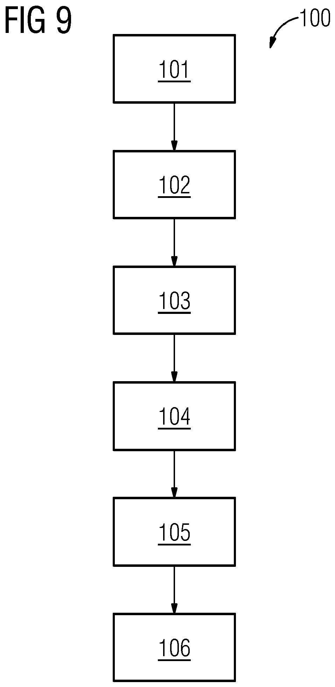

Die Fig. 9 zeigt eine beispielhafte Ausführung des erfindungsgemäßen Verfahrens 100. Das Verfahren 100 zur automatischen Positionierung einer Röntgenquelle 3 eines medizinischen Röntgensystems mit einem mobilen Röntgendetektor 5 weist die Schritte des Bestimmens 101, des Erfassens 102, des Lokalisierens 103, des Ermittelns 104 und des automatischen Positionierens 105 auf.

Im Schritt des Bestimmens 101 wird eine Untersuchungsregion des Untersuchungsobjekts bestimmt. Im Schritt des Erfassens 102 wird eine Position und eine Lage des Untersuchungsobjekts und die Untersuchungsregion mittels eines optischen Positionsbestimmungssystems erfasst. Im Schritt des Lokalisierens 103 wird die Untersuchungsregion lokalisiert. Im Schritt des Ermittelns 104 werden ein Aufpunkt des Zentralstrahls der Röntgenquelle und eine Blendengröße der Röntgenquelle basierend auf der lokalisierten Untersuchungsregion ermittelt. Im Schritt des automatischen Positionierens 105 wird die Röntgenquelle basierend auf dem Aufpunkt und der Blendengröße positioniert. Es kann ein weiterer Schritt des Anzeigens 106 auf einer Anzeigeeinheit erfolgen. Ferner kann der Schritt des Aufnehmens einer Röntgenaufnahme mit der positionierten Röntgenquelle erfolgen.

Der Schritt des Lokalisierens 103 kann folgende Schritte umfassen: den Schritt des Erzeugens eines Patientenmodells des Untersuchungsobjekts basierend auf der Erfassung des Untersuchungsobjekts mittels des optischen Positionsbestimmungssystems, den Schritt des Segmentieren des Patientenmodells basierend auf der bestimmten Untersuchungsregion, und den Schritt des Lokalisierens der Untersuchungsregion im Patientenmodell.

In einer anderen Ausführungsform kann der Schritt des Lokalisierens 103 folgende Schritte umfassen: den Schritt des Erzeugens eines Patientenmodells des Untersuchungsobjekts oder eines Bilddatensatzes des Untersuchungsobjekts basierend auf der Erfassung des Untersuchungsobjekts mittels des optischen Positionsbestimmungssystems, und den Schritt des Lokalisieren der Untersuchungsregion mittels einem trainierten Auswertungsverfahren basierend auf einem maschinellen Lernverfahren. Der Bilddatensatz kann insbesondere mittels des optischen Positionsbestimmungssystems erzeugt werden. Zur Auswertung des Bilddatensatzes können bekannte Bilderkennungsverfahren verwendet werden.

Eine Lage des Röntgendetektors wird basierend auf der Lage des Untersuchungsobjekts, des Patientenmodells oder des lokalisierten Untersuchungsbereichs ermittelt. Die Lage des Röntgendetektors kann, ggf. zusätzlich, mittels Winkelsensoren des Röntgendetektors ermittelt werden.

Eine Bewegung des Untersuchungsobjekts kann mittels des optischen Positionsbestimmungssystems erfasst werden. Eine erneute Erfassung und eine erneute Lokalisierung der Untersuchungsregion können durchgeführt werden und die Positionierung der Röntgenquelle kann basierend auf der erneuten Lokalisierung korrigiert werden.

Im Schritt des Anzeigens 106 kann der lokalisierte Untersuchungsbereich auf einer Anzeigeeinheit oder mittels eines auf das Untersuchungsobjekt projizierten Lichtfelds angezeigt werden.

Der Zentralstrahl der Röntgenquelle wird basierend auf der Untersuchungsregion oder einer Untersuchungsart in einem vorbestimmten Winkel zum Untersuchungsregion ausgerichtet. Der Zentralstrahl der Röntgenquelle kann im Wesentlichen senkrecht zum Röntgendetektor ausgerichtet werden. Alternativ kann der Zentralstrahl der Röntgenquelle von einer im Wesentlichen senkrechten Ausrichtung zum Röntgendetektor abweichen und eine Bildkorrektur einer Röntgenaufnahme kann hinsichtlich der Abweichung durchgeführt werden. Ferner kann ein Vorschlag zur Positionskorrektur des Untersuchungsobjekts, insbesondere an der Anzeigeeinheit, ausgegeben werden.

Obwohl die Erfindung im Detail durch das bevorzugte Ausführungsbeispiel näher illustriert wurde, so ist die Erfindung nicht durch die offenbarten Beispiele eingeschränkt und andere Variationen können vom Fachmann hieraus abgeleitet werden, ohne den Schutzumfang der Erfindung zu verlassen.

**Patentansprüche**

1. Verfahren (100) zur automatischen Positionierung einer Röntgenquelle (3) eines medizinischen Röntgensystems mit einem mobilen Röntgendetektor (5) aufweisend 2- P die computer-implementierten Schritte:

   a. Bestimmen (101) einer Untersuchungsregion (7) des Untersuchungsobjekts (20),
   b. Erfassen (102) einer Position und einer Lage des Untersuchungsobjekts und der Untersuchungsregion mittels eines optischen Positionsbestimmungssystems,
   c. Lokalisieren (103) der Untersuchungsregion,
   d. Ermitteln (104) eines Aufpunkts (9) des Zentralstrahls (4) der Röntgenquelle und einer Blendengröße der Röntgenquelle basierend auf der lokalisierten Untersuchungsregion, und
   e. automatisches Positionieren (105) der Röntgenquelle basierend auf dem Aufpunkt und der Blendengröße.

2. Verfahren nach Anspruch 1, wobei im Schritt des Lokalisierens folgende Schritte umfasst sind:

   i. Erzeugen eines Patientenmodells des Untersuchungsobjekts basierend auf der Erfassung des Untersuchungsobjekts mittels des optischen Positionsbestimmungssystems,
   ii. Segmentieren des Patientenmodells basierend auf der bestimmten Untersuchungsregion,
   iii. Lokalisieren der Untersuchungsregion im Patientenmodell.

3. Verfahren nach Anspruch 1, wobei im Schritt des Lokalisierens folgende Schritte umfasst sind:

   i. Erzeugen eines Patientenmodells des Untersuchungsobjekts oder eines Bilddatensatzes des Untersuchungsobjekts basierend auf der Erfassung des Untersuchungsobjekts mittels des optischen Positionsbestimmungssystems,
   ii. Lokalisieren der Untersuchungsregion mittels einem trainierten Auswertungsverfahren basierend auf einem maschinellen Lernverfahren.

4. Verfahren nach einem der vorangehenden Ansprüche, wobei eine Lage des Röntgendetektors basierend auf der Lage des Untersuchungsobjekts, des Patientenmodells oder des lokalisierten Untersuchungsbereichs ermittelt wird.

5. Verfahren nach einem der vorangehenden Ansprüche, wobei die Lage des Röntgendetektors mittels Winkelsensoren des Röntgendetektors ermittelt wird.

6. Verfahren nach einem der vorangehenden Ansprüche, wobei eine Bewegung des Untersuchungsobjekts mittels des optischen Positionsbestimmungssystems erfasst wird.

7. Verfahren nach Anspruch 6, wobei eine erneute Erfassung und eine erneute Lokalisierung der Untersuchungsregion durchgeführt wird und die Positionierung der Röntgenquelle basierend auf der erneuten Lokalisierung korrigiert wird.

8. Verfahren nach einem der vorangehenden Ansprüche, wobei der lokalisierte Untersuchungsbereich auf einer Anzeigeeinheit oder mittels eines auf das Untersuchungsobjekt projizierten Lichtfelds angezeigt wird.

9. Verfahren nach einem der vorangehenden Ansprüche, wobei der Zentralstrahl der Röntgenquelle basierend auf der Untersuchungsregion oder einer Untersuchungsart in einem vorbestimmten Winkel zum Untersuchungsregion ausgerichtet wird.

10. Verfahren nach einem der vorangehenden Ansprüche, wobei der Zentralstrahl der Röntgenquelle im Wesentlichen senkrecht zum Röntgendetektor ausgerichtet wird.

11. Verfahren nach einem der Ansprüche 1 bis 9, wobei der Zentralstrahl der Röntgenquelle von einer im Wesentlichen senkrechten Ausrichtung zum Röntgendetektor abweicht und eine Bildkorrektur einer Röntgenaufnahme hinsichtlich der Abweichung durchgeführt wird.

12. Verfahren nach einem der vorangehenden Ansprüche, wobei ein Vorschlag zur Positionskorrektur des Untersuchungsobjekts ausgegeben wird.

13. Röntgensystem adaptiert zum Durchführen eines Verfahrens nach einem der vorangehenden Ansprüche aufweisend:

   a. eine Röntgenquelle mit einem optischen Positionsbestimmungssystem, und
   b. einen mobilen Röntgendetektor, und
   c. Mittel zum automatischen Verfahren der Röntgenquelle.

14. Computerprogrammprodukt mit einem Computerprogramm, welches direkt in eine Speichereinrichtung einer Steuereinrichtung eines Röntgensystems ladbar ist, mit Programmabschnitten, um alle Schritte eines Verfahrens nach einem der Ansprüche 1 bis 12 auszuführen, wenn das Computerprogramm in der Steuereinrichtung des Röntgensystems ausgeführt wird.

15. Computerlesbares Medium, auf welchem von einer Rechnereinheit einlesbare und ausführbare Programmabschnitte gespeichert sind, um alle Schritte eines Verfahrens nach einem der Ansprüche 1 bis 12 auszuführen, wenn die Programmabschnitte von dem Röntgensystem ausgeführt werden.

**Claims**

1. Method (100) for automatic positioning of an X-ray source (3) of a medical X-ray system with a mobile X-ray detector (5) having the computer-implemented steps:

   a. determining (101) an examination region (7) of the examination object (20),
   b. acquiring (102) a position and a location of the examination object and the examination region by means of an optical position determining system,
   c. localising (103) the examination region,
   d. ascertaining (104) a field point (9) of the central ray (4) of the X-ray source and a collimator size of the X-ray source on the basis of the localised examination region, and
   e. automatic positioning (105) of the X-ray source on the basis of the field point and the collimator size.

2. Method according to claim 1, wherein in the step of localising, the following steps are included:

   i. creating a patient model of the examination object on the basis of the acquisition of the examination object by means of the optical position determining system,
   ii. segmenting the patient model on the basis of the determined examination region,
   iii. localising the examination region in the patient model.

3. Method according to claim 1, wherein in the step of localising, the following steps are included:

   i. creating a patient model of the examination object or an image data set of the examination object on the basis of the acquisition of the examination object by means of the optical position determining system,
   ii. localising the examination region by means of a trained evaluating method on the basis of a machine learning method.

4. Method according to one of the preceding claims, wherein a location of the X-ray detector is ascertained on the basis of the location of the examination object, the patient model or the localised examination field.

5. Method according to one of the preceding claims, wherein the location of the X-ray detector is ascertained by means of angle sensors of the X-ray detector.

6. Method according to one of the preceding claims, wherein a movement of the examination object is acquired by means of the optical position determining system.

7. Method according to claim 6, wherein a renewed acquisition and a renewed localisation of the examination region is carried out and the positioning of the X-ray source is corrected on the basis of the renewed localisation.

8. Method according to one of the preceding claims, wherein the localised examination field is displayed on a display unit or by means of a light field projected onto the examination object.

9. Method according to one of the preceding claims, wherein the central ray of the X-ray source is aligned at predetermined angle to the examination region, on the basis of the examination region or an examination type.

10. Method according to one of the preceding claims, wherein the central ray of the X-ray source is aligned substantially perpendicular to the X-ray detector.

11. Method according to one of claims 1 to 9, wherein the central ray of the X-ray source deviates from an alignment substantially perpendicular to the X-ray detector and an image correction of an X-ray recording is carried out in respect of the deviation.

12. Method according to one of the preceding claims, wherein a proposal for position correction of the examination object is output.

13. X-ray system adapted for carrying out a method according to one of the preceding claims, having:

   a. an X-ray source with an optical position determining system and
   b. a mobile X-ray detector and
   c. means for the automatic movement of the X-ray source.

14. Computer program product having a computer program which can be loaded directly into a memory apparatus of a control apparatus of an X-ray system, having program portions in order to carry out all the steps of a method according to one of claims 1 to 12 when the computer program is executed in the control apparatus of the X-ray system.

15. Computer-readable medium on which program portions that are configured to be read in and executed by a computer unit are stored, in order to carry out all the steps of a method according to one of claims 1 to 12 when the program portions are executed by the X-ray system.

**Revendications**

1. Procédé (100) de positionnement automatique d'une source (3) de rayons X d'un système de rayons X médical, comprenant un détecteur (5) mobile de rayons X, comportant les stades mis en œuvre par ordinateur :

    a. définition (101) d'une région (7) à examiner de l'objet (20) à examiner,
    b. relevé (102) d'une position et d'une situation de l'objet à examiner et de la région à examiner au moyen d'un système optique de définition de position,
    c. localisation (103) de la région à examiner,
    d. détermination (104) d'un point (9) d'incidence du rayon (4) central de la source de rayons X et d'une grandeur de diaphragme de la source de rayons X sur la base de la région à examiner localisée, et
    e. positionnement (105) automatique de la source de rayons X sur la base du point d'incidence et de la grandeur de diaphragme.

2. Procédé suivant la revendication 1, dans lequel, dans le stade de la localisation sont compris les stades suivants :

    i. production d'un modèle de patient de l'objet à examiner, sur la base du relevé de l'objet à examiner au moyen du système optique de définition de position,
    ii. segmentation du modèle de patient reposant sur la région définie à examiner,
    iii. localisation de la région à examiner dans le modèle de patient.

3. Procédé suivant la revendication 1, dans lequel dans la localisation sont compris les stades suivants :

    i. production d'un modèle de patient de l'objet à examiner ou d'un ensemble de données d'image de l'objet à examiner, sur la base du relevé de l'objet à examiner au moyen du système optique de définition de position,
    ii. localisation de la région à examiner au moyen d'un procédé d'analyse ayant subi un apprentissage sur la base d'un procédé d'apprentissage automatique.

4. Procédé suivant l'une des revendications précédentes, dans lequel on détermine une situation du détecteur de rayons X, sur la base de la situation de l'objet à examiner, du modèle de patient ou de la région à examiner localisée.

5. Procédé suivant l'une des revendications précédentes, dans lequel on détermine la situation du détecteur de rayons X au moyen de capteurs d'angle du détecteur de rayons X.

6. Procédé suivant l'une des revendications précédentes, dans lequel on relève un mouvement de l'objet à examiner au moyen du système optique de définition de position.

7. Procédé suivant la revendication 6, dans lequel on effectue un nouveau relevé et une nouvelle localisation de la région à examiner et on corrige le positionnement de la source de rayons X, sur la base de la nouvelle localisation.

8. Procédé suivant l'une des revendications précédentes, dans lequel on indique la région à examiner localisée sur une unité d'indication ou au moyen d'un champ lumineux projeté sur l'objet à examiner.

9. Procédé suivant l'une des revendications précédentes, dans lequel on dirige, suivant un angle défini à l'avance par rapport à la région à examiner, le rayon central de la source de rayons X, sur la base de la région à examiner ou d'un type d'examen.

10. Procédé suivant l'une des revendications précédentes, dans lequel on dirige le rayon central de la source de rayons X sensiblement perpendiculairement au détecteur de rayons X.

11. Procédé suivant l'une des revendications 1 à 9, dans lequel le rayon central de la source de rayons X s'écarte d'une direction sensiblement perpendiculaire au détecteur de rayons X et on effectue, en ce qui concerne l'écart, une correction d'image d'une radiographie.

12. Procédé suivant l'une des revendications précédentes, dans lequel on émet une proposition de correction de position de l'objet à examiner.

13. Système de rayons X propre à effectuer un procédé suivant l'une des revendications précédentes, comportant :

    a. une source de rayons X ayant un système optique de définition de position, et
    b. un détecteur mobile de rayons X, et

   c. des moyens de déplacement automatiques de la source de rayons X.

**14.** Produit de programme d'ordinateur ayant un programme d'ordinateur, qui peut être chargé directement dans un dispositif de mémoire d'un dispositif de commande d'un système de rayons X, comprenant des parties de programme pour effectuer tous les stades d'un procédé suivant l'une des revendications 1 à 12, lorsque le programme d'ordinateur est réalisé dans le dispositif de commande du système de rayons X.

**15.** Support déchiffrable par ordinateur, sur lequel sont mémorisées des parties de programme pouvant être déchiffrées et réalisées par une unité informatique, pour effectuer tous les stades suivant l'une des revendications 1 à 12, lorsque les parties de programme sont réalisées par le système de rayons X.

## FIG 1

## FIG 2

# FIG 3

# FIG 4

FIG 5

FIG 6

## FIG 7

20

20'

5

4

## FIG 8

3

40

4

31

20

32

5

30

FIG 9